# EUROPEAN PATENT SPECIFICATION

(11) **EP 4 661 829 B1**
(45) Date of publication and mention of the grant of the patent: **19.08.2026**
(21) Application number: 24701350.1
(22) Date of filing: 18.01.2024
(51) Int. Cl.: A61K 8/04, A61K 8/19, A61K 8/25, A61K 8/29, A61K 8/58, A61K 8/92, A61Q 17/04, A61K 8/26, A61K 8/891, A61K 8/895

(54) **WATER-FREE PERSONAL CARE COMPOSITION**
WASSERFREIE KÖRPERPFLEGEZUSAMMENSETZUNG
COMPOSITION DE SOINS PERSONNELS SANS EAU

(30) Priority: 07.02.2023 EP 23155351
(43) Date of publication of application: 17.12.2025
(73) Proprietor: Unilever IP Holdings B.V., 6708 WH Wageningen (NL); Unilever Global IP Limited, Wirral, Merseyside CH62 4ZD (GB)
(72) Inventor: JOSHI, Omkar Swapnil, 6708 WH Wageningen (NL); KULKARNI, Aditi Jayavant, 6708 WH Wageningen (NL); LAHORKAR, Praful Gulab Rao, 6708 WH Wageningen (NL); VAIDYA, Ashish Anant, 6708 WH Wageningen (NL); VARSHNEY, Jonish, 6708 WH Wageningen (NL)
(74) Representative: Unilever Patent Group
(86) International application number: PCT/EP2024/051108
(87) International publication number: WO 2024/165290

(56) References cited:
- EP-A1- 2 923 736
- WO-A1-2021/211468

## Description

### Field of the Invention

The present invention relates to a personal care composition that delivers effective sun protection through a topical composition that combines the pleasant sensorial of both a crème and a mousse type composition. The product thus delivers high SPF through a composition that gives a very light/fluffy, east to blend format with silky smooth end feel.

### Background of the Invention

Solar radiation includes about 5% ultraviolet (UV) radiation, wavelength of which is between 200 nm and 400 nm. It is further divided into three regions: from 320 to 400 nm (UV-A), 280 to 320 nm (UV-B) and from 200 to 280 nm (UV-C). Substantial portion of UV-C radiation is absorbed by ozone. Scientific studies have indicated that exposure to UV-A and UV-B radiation for short period causes pigmentation, reddening of the skin and localized irritation, whereas continued or prolonged exposure can lead to sunburn, melanoma, formation of wrinkles and age spots. UV radiation is also thought to cause significant damage to hair. Therefore, it is desirable to protect the skin and other keratinous substrates of the human body from the harmful effects of both UV-A and UV-B radiation. This is delivered though cosmetic compositions which provide high SPF (Sun Protection Factor).

Various cosmetic preparations are reported for preventing and/or protecting the skin from harmful effects of ultraviolet radiation. Numerous organic sunscreen agents capable of absorbing UV-A rays are reported in the field of cosmetics amongst which a particularly useful sunscreen is t-butylmethoxydibenzoylmethane (also known as Avobenzone and sold as Parsol^{®} 1789). A variety of UV-B sunscreens are used in personal care compositions for protection from UV-B radiation. Many cosmetic manufacturers prefer to include UV-A as well as UV-B sunscreens in photoprotective compositions to provide protection over the entire range of UV radiation.

These compositions are delivered through various types of products like creams, lotions, gels, serums, mousse etc. Each of these types of products have their own tactile sensory benefits. Not all sensorials are liked by everyone. The type of product used by a consumer depends on the person's skin type (dry, oily, sweaty etc.), on the weather conditions (dry, humid, cold or hot), type of cleansing composition used by the person etc. Cream compositions are available that can give a draggy matte feel that are liked by many consumers. Cream composition can also be tailored to give an oily feel which may be liked by consumers in very dry places especially in people with dry skin. Similar products are also available in lotion formats. Gel based compositions generally provide good moisturisation to the skin. In spite of very many products in various formats available in the market, there is still a need in the consumers to use products which are better liked as compared to the existing product types.

Two popular composition types are cremes and mousses. Crème is generally recognised as a liquid to creamy format which has low to medium coverage. Cremes generally comprise of silicone, fatty acid as oil emulsification agent, silicone elastomer, oils, hydrophobic pigment, sunscreen, electrolyte, actives etc. Actives and pigments are added give desired skin care benefits along with shade suitability. Cremes have both oil phase, aqueous phase and can be formulated as an oil in water or a water in oil emulsion. Sensorially they can be matt or velvety, and have moisturising capability and glossy look.

Mousse foundations are thicker in consistency than liquid foundation but is airy and super lightweight with velvety/feather like texture. They are anhydrous or water in oil emulsions (also known as the whipped foundation) and contains silicone oils, silicone elastomers, and other Ingredients. It usually comes in a pot or tube and is perfect for anyone looking for medium to high coverage, minus the cakey look. They are easy to spread and quickly blend with skin leaving a non-greasy skin feel.

In developing a product for sunscreen application which would deliver the desired SPF (sun protection factor) while ensuring a sensory feel which has the positives of both a crème and a mousse product, i.e. one that is very light and fluffy while being easy to blend with silky smooth end feel, the present inventors hit upon an anhydrous composition that has been rated by consumers highly on matiness, high oil control and liked by consumers who prefer a non-oily, non-sticky and non-greasy feel.

This has been achieved through a composition comprising very selective combination of a high amount of silicone elastomer, high amount of oil and very selective low amount of inorganic pigment. Composition comprising these ingredients have been reported in patent and open literature and products too are available in the market.

WO02/03935 (P&G) discloses an anhydrous skin treatment composition which includes a crosslinked siloxane elastomer gel of specific yield point, a skin conditioning agent and a volatile siloxane. Inclusions of the select elastomers provide improved uniform distribution of the pigments.

EP2923736 (J&J, 2015) discloses a method of making an anhydrous, pigmented sunscreen composition. The method comprises first adding a hydrophobic silica to a lipid phase comprising a dispersed color pigment and an organic UV filter and homogenizing the mixture. Next, a hydrophilic silica is added and rehomogenization is performed.

US2007/0297997 (P&G) discloses stable personal care composition comprising from about 1% to about 20% of a non-emulsifying silicone elastomer; from about 0.5% to about 10% of an emulsifying silicone elastomer; from about 5% to about 30% of an oil-soluble sunscreen; from about 1% to about 20% of a sunscreen solvent; and from about 30% to about 80% of a non-polar emollient.

WO 2021/211468 A1 refers to anhydrous silky cosmetic products comprising an anhydrous dimethicone crosspolymer gel dispersed in a first non-silicone fluid. These compositions do not comprise an inorganic pigment or a silicone elastomer at the claimed amount ranges.

While the above references disclose compositions that utilise the common ingredients used in the present invention they do not disclose the selective concentrations of the claimed ingredients in such a way that the desired tactile sensory feel on the skin are obtained.

It is thus an object of the present invention to deliver the desired very light/fluffy, east to blend format with silky smooth end feel from an anhydrous composition.

It is another object of the present invention to deliver high SPF from such a composition.

### Summary of the Invention

The present invention relates to a personal care composition comprising
(a) 40-60 wt% silicone elastomer;
(b) 25-35 wt% oil;
(c) 3-5 wt% inorganic pigment selected from one or both of titanium dioxide and mica, preferably titanium dioxide;
wherein the composition is substantially free of water.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The composition of the invention is meant to be used for personal care or for cosmetic use and could also be referred to as a personal care composition or a cosmetic composition. By a "personal care composition" as used herein, is meant to include a composition for topical application to sun-exposed areas of the skin and/or hair of humans. Such a composition is generally classified as leave-on composition, and includes any product applied to a human body for sun protection, improving appearance, odour control or general aesthetics.

"Skin" as used herein is meant to include skin on the face and body (e.g., neck, chest, back, arms, underarms, hands, legs and scalp) and especially to the sun exposed parts thereof. Compositions of the invention is also of relevance to applications on any other keratinous substrates of the human body other than skin e.g., hair where products may be formulated with specific aim of improving photoprotection.

The composition of the invention comprises a silicone elastomer included in 40 to 60 wt%, preferably from 45 to 55 wt%. The silicone elastomer is preferably selected from one or more of cyclopentasiloxane (and) dimethicone crosspolymer, dimethicone (and) vinyl dimethicone crosspolymer or dimethicone (and) cetearyl dimethicone crosspolymer; preferably cyclopentasiloxane (and) dimethicone crosspolymer. Cyclopentasiloxane is a 5 unit long, cyclic structured silicone that is volatile silicone but gives a smooth feel to hair and skin. It is often combined with non-volatile dimethicone for different skin or hair related benefits. The elastomeric mixture of high molecular weight silicone elastomers (dimethicone crosspolymer) in cyclopentasiloxane is available as DOWSIL^{™} 9045 Silicone Elastomer Blend. Dimethicone/vinyl dimethicone crosspolymer is a white silicone powder that gives skin a silky and smooth feel. It also known to absorbs excess oil and sebum from the skin and acts as a viscosity increasing agent. It is available as DOWSIL^{™} 9506 Powder.

Cetearyl dimethicone crosspolymer is an alkyl substituted dimethicone which functions to help increase organic compatibility in formulations. It has a high swelling capability in hydrocarbon oils and increases the viscosity of the oil phase. It is available as SeraSilk^{®} AM 76.

The composition of the invention comprises 25 to 35 wt% an oil. The oil may preferably be a silicone oil or an organic oil containing organic sunscreens.

A composition of the invention preferably comprises silicone oil in 20 - 30 wt% of the composition. Silicone oils may be divided into the volatile and nonvolatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 5 to 6, silicon atoms.

Nonvolatile silicone oils useful as emollient materials include polyalkyl siloxanes, polyalkylaryl siloxanes and polyether siloxane copolymers. The essentially nonvolatile polyalkyl siloxanes useful herein include, for example, polydimethyl siloxanes. Among the preferred nonvolatile oils useful in the present compositions are the polydimethyl siloxanes having viscosities from 1 x 10⁵ to 4 x 10⁴ m²/s at 25 °C.

The compositions of the invention may comprise an organic sunscreen. The organic sunscreen may be an oil soluble sunscreen or a water soluble sunscreen, preferably an oil soluble sunscreen. By oil-soluble is meant those organic sunscreens which have a solubility in water of less than 10 g/l at 25 °C. Oil-soluble sunscreens could be of the UV-B type or of the UV-A type.

When an oil soluble UVB sunscreen is included, it is preferably one or more of octyl salicylate, 3,3,5-trimethylclohexyl 2-hydroxybenzoate, ethylhexyl salicylate, 2-ethylhexyl 2-cyano-3,3-diphenyl-2-propenoate which is also known as octocrylene, 2-ethylhexyl-4-methoxycinnamate or octylmethoxycinnamate, ethylhexyl triazone, 3-benzylidene camphor, cinoxate, diethylhexyl butamido triazone, para amino benzoic acid (PABA), ethyl dihydroxypropyl PABA, ethylhexyl dimethoxy benzylidene dioxoimidazoline propionate, isoamyl p-methoxycinnamate, isopentyl trimethoxycinnamate trisiloxane, isopropyl benzyl salicylate, isopropyl methoxycinnamate, 4-methylbenzylidene camphor, PEG-25 PABA, and pentyl dimethyl PABA. More preferred oil-soluble UVB sunscreen is selected from one or more of octyl salicylate, 2-ethylhexyl 2-cyano-3,3-diphenyl-2-propenoate, and octylmethoxycinnamate.

Some of the well-known brands under which the above sunscreens are sold are Octisalate^{®}, Homosalate^{®}, Neo Heliopan^{®}, Neo Heliopan^{®} AV, Neo Heliopan^{®} OS, Octocrylene^{®} and Parsol^{®} MCX. The oil-soluble UV-B sunscreen has λmax from 280 to 320 nm.

Composition of the invention may comprise an organic oil-soluble UVA sunscreen. When included, it is selected from one or more of a dibenzoylmethane compound, diethylamino hydroxybenzoyl hexyl benzoate and methyl anthranilate. A dibenzoyl methane compound is most preferred for an oil-soluble UVA-sunscreen. Suitable dibenzoyl methane compounds are selected from one of more of t-butylmethoxy dibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane and 2,6-dimethyl-4-tert-butyl-4'-methoxydibenzoylmethane. The most preferred dibenzoyl methane compound is t-butylmethoxy dibenzoylmethane. Commercially available as Parsol 1789.

Composition of the invention may comprise both an organic oil soluble UVB and an organic UVA sunscreen. Alternately, there are certain oil soluble sunscreens which have both a UVA and UVB screening efficacy. It is selected from one or more bis-ethylhexyloxyphenol methoxyphenyl triazine, drometrizole trisiloxane, benzophenone and its derivatives. The most preferred one is bis-ethylhexyloxyphenol methoxyphenyl triazine, commercially available as Tinosorb S.

The composition of the invention may also comprise a water-soluble sunscreen. By water soluble sunscreen is meant that the solubility in water of the sunscreen is higher than 10 g/l at 25 °C.

The preferred water-soluble sunscreens are phenyl benzimidazole 5-sulphonic acid (PBSA), benzophenone-4 or benzylidene camphor sulfonic acid, preferably PBSA. PBSA also known as Ensulizole is commercially available as Eusolex 232 (from Merck KGaA). PBSA is also available under the brand names Neo Heliopan Hydro (from Symrise), Parsol HS (from DSM) and Sunsafe ES (from Uniproma). Alternately the water soluble sunscreen may be benzophenone-4 (sold as Sulisobenzone). Other preferred water soluble sunscreens are disodium phenyl dibenzimidazole tetrasulfonate ( available as Neoheliopan AP from Symrise Shanghai Ltd or as Sunsafe DPDT from Uniproma) or terephthalylidene dicamphor sulfonic acid (TDSA). TDSA is also known as Ecamsule . This is commercially available as Mexoryl SX (US4585597) by L' Oreal or Sunsafe TDSA (from Uniproma).

Preferably, the composition may comprise an inorganic sunscreen. Example of inorganic sunscreen that may be used in the composition is zinc oxide. When incorporated in the composition, the inorganic sunscreen may be included in 5 to 15, more preferably from 6 to 12% by weight of the composition.

The composition may additionally include an inorganic particle. Inorganic particle may be selected from one or more of iron oxide, talc, starch and it derivatives and silica. The inorganic particle is preferably one or more of iron oxide, talc, and silica. When included, it is present in 1 to 5%, preferably 1.5 to 4% by weight of the composition.

The composition of the invention is an anhydrous composition and therefore is substantially free of water. By substantially free of water means that no water is intentionally included in the composition for any functional benefit. Any water that is present is by way of water present in the raw materials as a consequence of its being in equilibrium with the humidity of the ambient air. In consequence, the amount of water is preferably at most in the range of 0.1 to 4 wt%, more preferably in the range of 0.1 to 3wt%. The composition preferably is substantially free of a surfactant. By substantially free of a surfactant is meant that a surfactant is not intentionally included for any functional purpose. It is generally expected that a surfactant, if any is present in less than 1 wt%, preferably less than 0.5wt%, more preferably less than 0.1 wt% and optimally absent from the composition.

The composition of the invention also delivers as good SPF and UVA protection factor (UVAPF) values for the same amount of sunscreens as delivered through a conventional cream composition. Further the present invention also delivers broad spectrum protection. This is evident from the CW values measured for the compositions. CW value is the Critical wavelength value.

Critical wavelength is the wavelength that determines the broad spectrum of sunscreens and in order to qualify as broad spectrum, 90% or higher amount of the rays should be absorbed in a wavelength of 370 nm or above. The US FDA for the first time issued an in vitro pass/fail test for sunscreen efficacy testing in the monograph-2011. The purpose was to determine if the UV protection of the particular sunscreen formulation can be considered "broad spectrum." The critical wavelength of a sunscreen needs to be equal to or higher than 370 nm in order to claim "broad spectrum". Only "broad spectrum" sunscreens with an SPF equal to or higher than 15 can claim benefits against skin cancer and skin aging as directed in the monograph.

The invention will now be illustrated with the help of the following non-limiting examples.

### Examples

Examples A, B, 1: The following formulations were prepared.

Example A: A commercially available sunscreen cream was prepared as shown in Table -1:

**Table -1:**

| Ingredient | Function | Wt% |
|---|---|---|
| Cyclopentasiloxane (and) dimethicone crosspolymer | Silicone elastomer | 22.0 |
| Ethylhexyl methoxycinnamate | Oil; Organic sunscreen | 7.5 |
| Titanium dioxide | Inorganic pigment | 7.0 |
| Zinc Oxide | Inorganic sunscreen | 8.4 |
| Iron oxide | Inorganic particle | 1.4 |
| Niacinamide | Vitamin | 3.0 |
| Glycerine | Humectant | 3.0 |
| PEG-10 Dimethicone | Conditioning agent | 0.8 |
| Dicaprylyl carbonate | Conditioning agent; Emollient | 1.5 |
| Disteardimonium Hectorite | Thickener | 0.3 |
| Potassium chloride | Salt; Viscosity modifier | 2.0 |
| Water and minors | | To 100 |

Example B, 1: Anhydrous compositions as shown in Table - 2 were prepared.

**Table - 2:**

| Ingredient | Function | Example- B, Wt% | Example- 1, Wt% |
|---|---|---|---|
| Cyclopentasiloxane (and) dimethicone crosspolymer | Silicone elastomer | 50.7 | 49.2 |
| Cyclopentasiloxane | Oil | 26.5 | 25.0 |
| Cetyl dimethicone | Oil | 1.0 | 1.0 |
| Ethylhexyl methoxycinnamate | Oil; Organic sunscreen | 4.0 | 4.0 |
| Titanium dioxide | Inorganic pigment | 1.0 | 4.0 |
| Zinc Oxide | Inorganic sunscreen | 7.0 | 7.0 |
| Dimethicone/ Vinyl dimethicone crosspolymer (and) Silica | Inorganic particle | 4.0 | 4.0 |
| Silica | Inorganic particle | 0.7 | 0.7 |
| Talc | Inorganic particle | 1.8 | 1.8 |
| Iron oxide | Inorganic particle | 0.3 | 0.3 |
| Methyl Methacrylate/ IsoButylmethacrylate Crosspolymer | Film forming agents Thickener | 1.0 | 1.0 |
| PEG-10 Dimethicone | Conditioning agent | 0.2 | 0.2 |
| Parabens | Preservative | 0.6 | 0.6 |
| Perfume and minors | | 1.2 | 1.2 |

The compositions as per example A, B and 1 were evaluated by consumers using the following method.

The compositions were tested by consumers (N of about 70 in the age group of 18 to 30). The respondents were recruited offline via face-to-face interviews and recall was completed by filling online survey link, following a structured quantitative questionnaire.

The product was distributed in two different colour shades (Beige and Bronze) as per the consumer's skin tone and each respondent used the product over a span of 7 days prior to providing their opinion.

The consumers rated the products for the following parameters: Matiness, Oil control and Feel (oily/ sticky/ greasy feel) on the face post application. The data is summarised in Table -3 below:

**Table - 3**

| Example | Average rating on matiness | Average rating on Oil control | Feel on the face post application |
|---|---|---|---|
| A | 70.0 | 80.0 | 83.3 |
| B | 76.7 | 83.3 | 86.7 |
| 1 | 86.7 | 86.7 | 100 |

The data in the table - 3 above indicates that the composition as per the invention (Example -1) is better liked by consumers on all the measured sensory attributes as compared to a similar anhydrous composition outside the invention (Example B) and a commercially available cream composition (Example A).

### Sun protection (SPF) delivered by the compositions of Examples C,B,1:

Example C similar to Example A was prepared except that the amount of sunscreen agents was kept the same as in Example -1. Composition C is as given below in Table - 4.

**Table -4: Example C**

| Ingredient | Function | Wt% |
|---|---|---|
| Cyclopentasiloxane (and) dimethicone crosspolymer | Silicone elastomer | 22.0 |
| Ethylhexyl methoxycinnamate | Oil; Organic sunscreen | 4 |
| Titanium dioxide | Inorganic pigment | 4 |
| Zinc Oxide | Inorganic sunscreen | 7 |
| Iron oxide | Inorganic particle | 0.3 |
| Niacinamide | Vitamin | 3.0 |
| Glycerine | Humectant | 3.0 |
| PEG-10 Dimethicone | Conditioning agent | 0.8 |
| Dicaprylyl carbonate | Conditioning agent; Emollient | 1.5 |
| Disteardimonium Hectorite | Thickener | 0.3 |
| Potassium chloride | Salt; Viscosity modifier | 2.0 |
| Water and minors | | To 100 |

The in vitro SPF, UVA protection factor (UVAPF) and CW (critical wavelength) values for the compositions B, C and 1 were measured using the following procedure.

Thin film transmittance measurements were done using Labsphere's Ultraviolet transmittance analyzer. In this study, 70 x 70 mm PMMA plate with ~6 µm roughness from Schönberg GmbH & Co were used. The percent transmittance of the various compositions was measured using a procedure as outlined below. 2 mg/cm² of sample was applied on PMMA plate, distributed uniformly using a syringe, and spread uniformly. The drying time for the PMMA plates was 30 minutes. After the drying time, sample plates were exposed to UV light and transmittance scan was recorded. This scan gives the transmittance as a function of wavelength (290 - 400 nm) for a given sample. For a single plate six to nine different spots were scanned. The same was repeated for 2 or 3 plates. The data reported is thus an average over 12 -18 readings. The reference transmittance scan was obtained using a blank plate, with no sample on the PMMA plates with glycerine spread on it. The transmittance values were used to arrive at the SPF, the UVAPF and the CW values using the UV -2000s application provided with the instrument.

The invitro SPF, UVAPF along with the respective standard deviations and CW values of the various compositions is summarized in Table - 5:

**Table - 5:**

| N=2 (average of two PMMA Plates) | | | | | |
|---|---|---|---|---|---|
| Example | In vitro SPF | STDV | UVA PF | STDV | CW |
| Control | 1.00 | 0.01 | 0.0 | | |
| C | 21.26 | 6.96 | 7.7 | 1.27 | 382 |
| B | 18.07 | 5.16 | 8.17 | 2.27 | 382 |
| 1 | 23.94 | 4.63 | 8.69 | 1.725 | 382 |

The data in the table - 5 above indicates that composition as per the invention (Example -1) delivers similar, if not slightly better sun protection as compared to similar existing cream composition (Example C) and an anhydrous composition outside the present invention (Example - B). Further all the compositions can be considered broad spectrum, going by the measured CW values.

## Claims

1. A personal care composition comprising
(a) 40-60 wt% silicone elastomer;
(b) 25-35 wt% oil;
(c) 3-5 wt% inorganic pigment selected from one or both of titanium dioxide and mica, preferably titanium dioxide;
wherein the composition is substantially free of water.

2. A composition as claimed in claim 1 wherein the silicone elastomer is selected from one or more of cyclopentasiloxane (and) dimethicone crosspolymer, dimethicone (and) vinyl dimethicone crosspolymer or dimethicone (and) cetearyl dimethicone crosspolymer.

3. A composition as claimed in claim 1 or 2 wherein the oil is selected from one or both of a silicone oil or an organic oil containing organic sunscreen.

4. A composition as claimed in claim 3 wherein the silicone oil is included in 20 - 30 wt% of the composition.

5. A composition as claimed in claim 3 or 4 wherein the organic sunscreen is included in 1 to 8 wt% of the composition.

6. A composition as claimed in any one of the preceding claims additionally comprising 5 - 15 wt% of an inorganic sunscreen, preferably zinc oxide.

7. A composition as claimed in any one of the preceding claims additionally comprising 1 - 5 wt% of an inorganic particle selected from one or more of iron oxide, talc, starch and it's derivatives and silica.

8. A composition as claimed in any one of the preceding claims substantially free of a surfactant.

## Patentansprüche

1. Körperpflegezusammensetzung, umfassend
• (a) 40-60 Gew.-% Silikonelastomer;
• (b) 25-35 Gew.-% Öl;
• (c) 3-5 Gew.-% anorganisches Pigment, ausgewählt unter Titandioxid und/oder Glimmer, vorzugsweise Titandioxid:
wobei die Zusammensetzung im Wesentlichen frei von Wasser ist.

2. Zusammensetzung, wie im Anspruch 1 beansprucht, wobei das Silikonelastomer ausgewählt ist unter einem oder mehreren von Cyclopentasiloxan(und)Dimethicon-Crosspolymer, Dimethicon(und)Vinyl-dimethicon-Crosspolymer oder Dimethicon(und)Cetearyl-dimethicon-Crosspolymer.

3. Zusammensetzung, wie im Anspruch 1 oder 2 beansprucht, wobei das Öl unter einem Silikonöl und/oder einem organischen Öl, das ein organisches Sonnenschutzmittel enthält, ausgewählt ist.

4. Zusammensetzung, wie im Anspruch 3 beansprucht, wobei das Silikonöl in einer Menge von 20 bis 30 Gew.-% der Zusammensetzung vorliegt.

5. Zusammensetzung, wie im Anspruch 3 oder 4 beansprucht, wobei das organische Sonnenschutzmittel in einer Menge von 1 bis 8 Gew.-% der Zusammensetzung vorliegt.

6. Zusammensetzung, wie in irgendeinem der vorhergehenden Ansprüche beansprucht, die zusätzlich 5 bis 15 Gew.-% eines anorganischen Sonnenschutzmittels, vorzugsweise Zinkoxid, umfasst.

7. Zusammensetzung, wie in irgendeinem der vorhergehenden Ansprüche beansprucht, die zusätzlich 1 bis 5 Gew.-% eines anorganischen Partikels umfasst, ausgewählt unter einem oder mehreren von Eisenoxid, Talk, Stärke und deren Derivaten sowie Siliciumdioxid.

8. Zusammensetzung, wie in irgendeinem der vorhergehenden Ansprüche beansprucht, die im Wesentlichen frei von Tensid ist.

## Revendications

1. Composition de soins personnels comprenant
(a) 40 à 60 % en poids d'élastomère de silicone;
(b) 25 à 35 % en poids d'huile;
(c) 3 à 5 % en poids de pigment inorganique choisi parmi l'un ou les deux de dioxyde de titane et de mica, de préférence le dioxyde de titane;
où la composition est sensiblement dépourvue d'eau.

2. Composition selon la revendication 1 dans laquelle l'élastomère de silicone est choisi parmi un ou plusieurs de polymère réticulé de cyclopentasiloxane (et) diméthicone, de polymère réticulé de diméthicone (et) vinyldiméthicone ou de polymère réticulé de diméthicone (et) cétéaryldiméthicone.

3. Composition selon la revendication 1 ou 2 dans laquelle l'huile est choisie parmi l'une ou les deux d'une huile de silicone ou d'une huile organique contenant un filtre solaire organique.

4. Composition selon la revendication 3 dans laquelle l'huile de silicone est incluse à raison de 20 à 30 % en poids de la composition.

5. Composition selon la revendication 3 ou 4 dans laquelle le filtre solaire organique est inclus à raison de 1 à 8 % en poids de la composition.

6. Composition selon l'une quelconque des revendications précédentes comprenant en outre 5 à 15 % en poids d'un filtre solaire inorganique, de préférence l'oxyde de zinc.

7. Composition selon l'une quelconque des revendications précédentes comprenant en outre 1 à 5 % en poids d'une particule inorganique choisie parmi un ou plusieurs de l'oxyde de fer, le talc, l'amidon et ses dérivés et la silice.

8. Composition selon l'une quelconque des revendications précédentes sensiblement exempte de tensioactif.
